# EUROPÄISCHE PATENTSCHRIFT

(11) **EP 1 727 792 B1**
(45) Veröffentlichungstag und Bekanntmachung des Hinweises auf die Patenterteilung: **17.12.2008**
(21) Anmeldenummer: 05716121.8
(22) Anmeldetag: 16.03.2005
(51) Int. Cl.: C07C 281/04, C07C 281/14

(54) **VERFAHREN ZUR HERSTELLUNG VON SEMICARBAZONEN**
METHOD FOR PRODUCING SEMICARBAZONES
PROCEDE POUR PRODUIRE DES SEMICARBAZONES

(30) Priorität: 17.03.2004 EP 04006413; 17.03.2004 DE 102004013083
(43) Veröffentlichungstag der Anmeldung: 06.12.2006
(73) Patentinhaber: BASF SE, 67056 Ludwigshafen (DE)
(72) Erfinder: ZIERKE, Thomas, 67459 Böhl-Iggelheim (DE); ENGEL, Stefan, 55268 Nieder-Olm (DE); OTTO, Peter, 67127 Rödersheim-Gronau (DE)
(74) Vertreter: Reitstötter - Kinzebach
(86) Internationale Anmeldenummer: PCT/EP2005/002802
(87) Internationale Veröffentlichungsnummer: WO 2005/090293

(56) Entgegenhaltungen:
- EP-A- 0 500 111

## Beschreibung

Die vorliegende Erfindung betrifft ein Verfahren zur Herstellung von SemicarbazonVerbindungen der Formel I 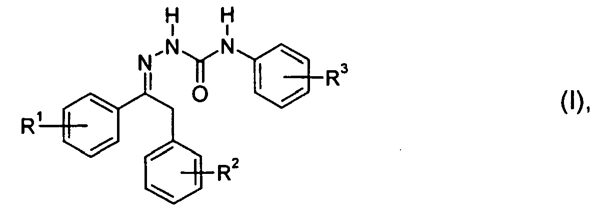 worin R¹ und R² unabhängig voneinander für Wasserstoff, Halogen, CN, C₁-C₄-Alkyl, C₁-C₄-Alkoxy, C₁-C₄-Haloalkyl oder C₁-C₄-Haloalkoxy stehen und R³ für C₁-C₄-Alkoxy, C₁-C₄-Haloalkyl oder C₁-C₄-Haloalkoxy steht.

Aus der EP-A 462 456 sind Verbindungen der Formel I als Insektizide bekannt. Ihre Herstellung erfolgt ausgehend von Benzylphenylketonen der Formel IV nach den in den Schemata 1 und 2 dargestellten Verfahren:

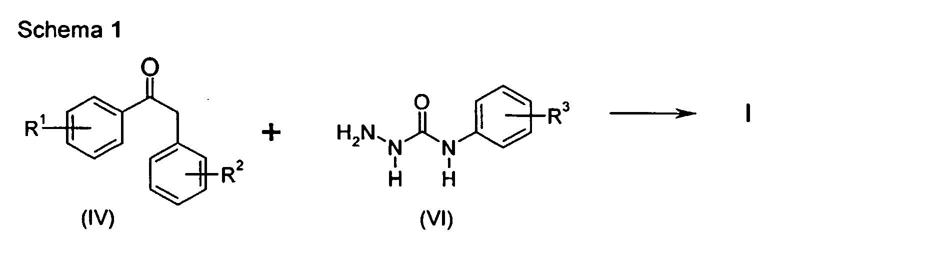

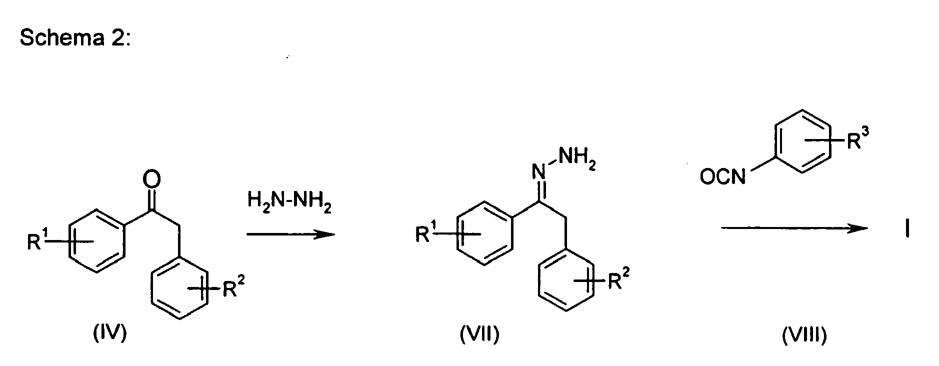

In den Schemata 1 und 2 haben R¹, R² und R³ die zuvor angegebenen Bedeutungen Bei der in Schema 1 dargestellten Umsetzung eines Phenylbenzylketons IV mit einem N-Phenylsemicarbazid VI wird die Verbindung I jedoch nur in schlechten Ausbeuten erhalten. Zudem muss das Semicarbazid VI in einem separaten Reaktionsschritt aus dem entsprechenden Anilin hergestellt werden.

Nachteilig an dem in Schema 2 dargestellten Verfahren ist zum einen der Einsatz von Hydrazin im ersten Reaktionsschritt, das zur Vermeidung von Nebenproduktbildung in hohem Überschuss eingesetzt werden muss. Bekanntermaßen handelt es sich bei Hydrazin um eine potentiell karzinogene Substanz, die zudem bei Kontakt mit metallischen Werkstoffen zur spontanen Zersetzung unter Gasbildung neigt. Der Umgang mit Hydrazin im Produktionsmaßstab ist daher aus sicherheitstechnischen Gründen nur mit einem sehr hohen technischen Aufwand möglich. Zudem ist die Entsorgung der bei dieser Reaktion anfallenden Hydrazinabfälle im industriellen Maßstab mit einem hohen Aufwand verbunden, da die chemische Vernichtung hochkonzentrierter Hydrazinlösungen stark exotherm verläuft und erhebliche Gasmengen freigesetzt werden. Der Einsatz von Hydrazin stellt somit einen erheblichen Kostenfaktor für dieses Verfahren dar. Ein weiterer Nachteil des in Schema 2 gezeigten Verfahrens ist der Einsatz von Phenylisocyanaten VIII, die zum einen aufgrund ihrer Toxizität besondere Sicherheitsmaßnahmen bei ihrer Handhabung erfordern und zudem in einer separaten Reaktion aus den entsprechenden Anilinen hergestellt werden müssen.

Der vorliegenden Erfindung liegt daher die Aufgabe zugrunde, ein Verfahren zur Herstellung von Semicarbazonverbindungen der allgemeinen Formel I bereitzustellen, das die Verbindung I in hohen Ausbeuten und guten Reinheiten liefert und das zudem die hier geschilderten Nachteile des Standes der Technik überwindet.

Es wurde überraschenderweise gefunden, dass Semicarbazonverbindungen der allgemeinen Formel I in guten Ausbeuten und mit hoher Reinheit erhalten werden, wenn man eine Hydrazonverbindung der allgemeinen Formel II, 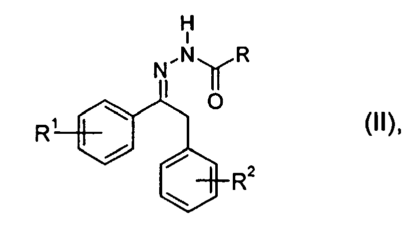 worin R für C₁-C₄-Alkoxy, Amino, C₁-C₄-Alkylamino oder Di-(C₁-C₄-alkyl)amino steht und R¹, R² die zuvor genannten Bedeutungen haben, mit einer Anilinverbindung der allgemeinen Formel III 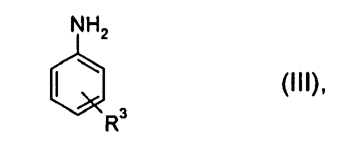 worin R³ die zuvor genannte Bedeutung hat, umsetzt.

Gegenstand der vorliegenden Erfindung ist daher ein Verfahren zur Herstellung von Semicarbazonverbindungen der allgemeinen Formel I, umfassend die Umsetzung einer Hydrazonverbindung der allgemeinen Formel II mit einem substituierten Anilin der Formel III.

Die Hydrazonverbindungen der allgemeinen Formel II können ihrerseits in Analogie zu bekannten Verfahren des Standes der Technik aus den Benzylphenylketonen der Formel IV 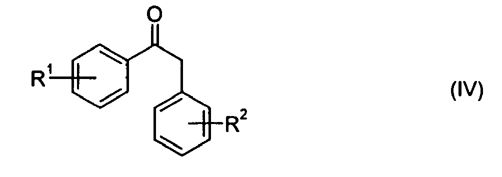 worin R¹, R² die zuvor genannten Bedeutungen aufweisen, durch Umsetzung von IV mit einem Hydrazid der Formel V 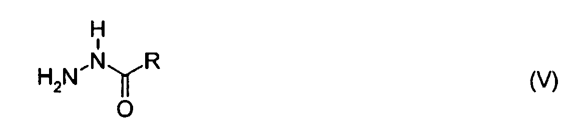 worin R die zuvor genannte Bedeutung hat, hergestellt werden. Demnach umfasst das erfindungsgemäße Verfahren vorzugsweise auch die Bereitstellung der Hydrazonverbindungen II auf diesem Wege.

Die Hydrazonverbindungen der Formel II sind für R² = CN neu und als Ausgangs- bzw. Zwischenprodukte im erfindungsgemäßen Verfahren ebenfalls Gegenstand der vorliegenden Erfindung.

Bei den in den vorstehenden Formeln angegebenen Definitionen der Variablen R, R¹, R² und R³ werden Sammelbegriffe verwendet, die allgemein repräsentativ für die jeweiligen Substituenten stehen. Die Bedeutung Cₙ-Cₘ gibt die jeweils mögliche Anzahl von Kohlenstoffatomen in dem jeweiligen Substituenten oder Substituententeil an. Im Übrigen bedeutet:
**Halogen:** Fluor, Chlor, Brom und Iod;
**Alkyl sowie alle Alkylteile in Alkoxy, Alkylamino und Dialkylamino:** gesättigte, geradkettige oder verzweigte Kohlenwasserstoffreste mit 1 bis 4 C-Atomen wie Methyl, Ethyl, Propyl, 1-Methylethyl, Butyl, 1-Methyl-propyl, 2-Methylpropyl und 1,1-Dimethylethyl (tert.-Butyl);
**Halogenalkyl sowie die Halogenalkylteile in Halogenalkoxy:** geradkettige oder verzweigte Alkylgruppen mit 1 bis 4 und insbesondere 1 oder 2 Kohlenstoffatomen (wie vorstehend genannt), wobei in diesen Gruppen teilweise oder vollständig die Wasserstoffatome durch Halogenatome wie vorstehend genannt und insbesondere durch Fluor (Fluoralkyl) ersetzt sein können, z. B. Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2,2,3,3,3-Pentafluorpropyl, Heptafluorpropyl und 1,1,1-Trifluorprop-2-yl. Bevorzugtes Halogenalkyl ist C₁-C₂-Fluoralkyl wie 2-Fluorethyl, 2,2-Difluorethyl, 2,2,2-Trifluorethyl, Trifluormethyl und Difluormethyl.

Für das erfindungsgemäße Verfahren hat es sich als besonders vorteilhaft erwiesen, wenn R in Formel II und dementsprechend auch in Formel V für C₁-C₄-Alkoxy, insbesondere Methoxy, steht.

In einer anderen Ausführungsform der Erfindung steht R für NH₂, C₁-C₄-Alkylamino oder Di-(C₁-C₄-alkyl)amino. R steht dann bevorzugt für Amino (NH₂), Methylamino, Ethylamino oder Dimethylamino und insbesondere für NH₂.

Die Vorteile des erfindungsgemäßen Verfahrens kommen insbesondere bei der Herstellung von Verbindungen der Formel I zum Tragen, worin die Variablen R¹, R² und R³ unabhängig voneinander und besonders bevorzugt gemeinsam die im Folgenden angegebenen Bedeutungen aufweisen:
- R¹: C₁-C₄-Halogenhalkyl, insbesondere Trifluormethyl und speziell Trifluormethyl, das in der meta-Position (3-Position) des Phenylrings angeordnet ist;
- R²: Cyano, insbesondere Cyano, das in der para-Position (4-Position) angeordnet ist;
- R³: C₁-C₄-Halogenalkoxy, insbesondere Trifluormethoxy und speziell Trifluormethoxy, das in der para-Position, angeordnet ist.

Zur Umsetzung von Anilinverbindung III mit dem Hydrazonderivat II wird man die Verbindungen vorzugsweise in einem Molverhältnis III:II im Bereich von 1:1,5 bis 1,5:1, insbesondere von 1:1 bis 1,3:1 und besonders bevorzugt von 1,02:1 bis 1,2:1 einsetzen.

Die Umsetzung der Anilinverbindung III mit der Hydrazonverbindung II erfolgt vorteilhafterweise bei Temperaturen oberhalb Raumtemperatur, z. B. im Bereich von 30 bis 200 °C, insbesondere 50 bis 180 °C und besonders bevorzugt im Bereich von 70 bis 150 °C. Der Reaktionsdruck ist für den Erfolg des erfindungsgemäßen Verfahrens von untergeordneter Bedeutung und kann beispielsweise im Bereich von 500 mbar bis 10 bar liegen. Vorzugsweise wird die Reaktion im Bereich des Normaldrucks, d. h. im Bereich von 0,9 bis 1,2 bar durchgeführt. Die für die Umsetzung erforderliche Reaktionszeit liegt in der Regel im Bereich von 1 h bis 24 h und insbesondere im Bereich von 3 h bis 12 h.

Die Umsetzung kann grundsätzlich in Substanz durchgeführt werden. Vorzugsweise setzt man jedoch die Anilinverbindung III mit der Hydrazonverbindung II in einem organischen Lösungsmittel um. Geeignet sind grundsätzlich alle Lösungsmittel, welche die Verbindungen II und III zumindest teilweise und vorzugsweise vollständig unter Reaktionsbedingungen zu lösen vermögen. Bevorzugte Lösungsmittel sind aprotisch. Insbesondere handelt es sich um solche Lösungsmittel, die bei Normaldruck einen Siedepunkt im Bereich von 60 bis 200 °C und insbesondere im Bereich von 80 bis 150 °C aufweisen. Besonders bevorzugte Lösungsmittel sind aromatische Lösungsmittel, insbesondere Alkylbenzole wie Toluol, Xylole, Ethylbenzol, Cumol (2-Propylbenzol), Cymole (Isopropyltoluole) und Mesitylen, sowie Chlorbenzole, z. B. Chlorbenzol, 1,2-, 1,3- und 1,4-Dichlorbenzol, weiterhin aliphatische Nitrile wie Acetonitril und Propionitril und Mischungen dieser Lösungsmittel.

Die Umsetzung der Anilinverbindung III mit der Hydrazonverbindung II kann in Gegenwart einer Säure durchgeführt werden, jedoch ist der Einsatz einer Säure nicht erforderlich. Beispiele prinzipiell geeigneter Säuren sind Schwefelsäure, organische Sulfonsäuren, insbesondere aromatische Sulfonsäuren wie p-Toluolsulfonsäure und Benzolsulfonsäure, aliphatische Sulfonsäuren wie Methansulfonsäure und Trifluormethansulfonsäure, aromatische Carbonsäuren wie Benzoesäure und 4-Trifluormethylbenzoesäure, sowie aliphatische Carbonsäuren mit vorzugsweise 1 bis 3 C-Atomen, z. B. Essigsäure und Propionsäure. In der Regel wird man die Säure in katalytischen Mengen, d. h. in Mengen von weniger als 1 mol pro Mol Verbindung II oder III, insbesondere weniger als 0,5 mol/mol und speziell nicht mehr als 0,2 mol/mol einsetzen. In einer bevorzugten Variante des erfindungsgemäßen Verfahrens erfolgt die Umsetzung von II mit III in Abwesenheit einer Säure.

Vorzugsweise wird man die bei der Umsetzung des Anilins II mit dem Hydrazon III gebildete Verbindung R-H zumindest teilweise, vorzugsweise zu wenigsten 50 % und insbesondere zu wenigstens 80 % während der Umsetzung aus der Reaktionsmischung entfernen, insbesondere wenn es sich bei der Verbindung R-H um ein C₁-C₄-Alkanol wie Methanol oder Ethanol handelt. Hierzu wird man in die Reaktion bei einer Temperatur und einem Druck durchführen, bei der die Verbindung R-H, gegebenenfalls als Azeotrop mit dem Lösungsmittel aus der Reaktionsmischung abdestilliert. Gegebenenfalls wird man zum Ausgleich frisches Lösungsmittel in die Reaktion einbringen oder das mit der Verbindung R-H abdestillierte Lösungsmittel nach ggf. destillativer Abreicherung der Verbindung R-H in die Reaktion zurückführen. Aus diesen Gründen ist es von Vorteil, wenn das eingesetzte Lösungsmittel einen Siedepunkt von wenigstens 10 K und insbesondere wenigstens 30 K oberhalb des Siedepunkts der bei der Reaktion gebildeten Verbindung R-H aufweist (jeweils bei Normaldruck). Zweckmäßigerweise führt man die Umsetzung der Verbindung II mit der Verbindung III in einer Apparatur durch, die mit wenigstens einer Destillations- und Rektifikationsvorrichtung, z. B. einer Destillationskolonne, versehen ist, welche zum einen ein Abdestillieren der Verbindung R-H, ggf. zusammen mit Lösungsmittel, erlaubt und gleichzeitig eine Abtrennung und Rückführung des gegebenenfalls mit der Verbindung R-H abdestillierten Lösungsmittels ermöglicht.

Zur Umsetzung können die Verbindungen II und III in an sich beliebiger Weise miteinander in Kontakt gebracht werden. Beispielsweise kann man die Verbindung II und III in einem Reaktionsgefäß, gegebenenfalls zusammen mit dem gewünschten Lösungsmittel, vorlegen und dann die gewünschten Reaktionsbedingungen einstellen. Man kann jedoch auch die Hauptmenge oder Gesamtmenge an Verbindungen II und III, gegebenenfalls in einem Lösungsmittel, unter Reaktionsbedingungen in das Reaktionsgefäß eintragen. In einer bevorzugten Ausführungsform der Erfindung legt man die Hauptmenge, insbesondere wenigstens 80 % und besonders bevorzugt die Gesamtmenge oder nahezu die Gesamtmenge (> 95 %) der Hydrazonverbindung II, gegebenenfalls in dem gewünschten Lösungsmittel, vor und gibt hierzu im Verlauf der Reaktion, z. B. über einen Zeitraum von 0,5 bis 20 h und insbesondere 1 bis 10 h, die Hauptmenge, insbesondere wenigstens 80 % und besonders bevorzugt die Gesamtmenge oder nahezu die Gesamtmenge (> 95 %) der Anilinverbindung unter Reaktionsbedingungen zu. Hierzu wird man die Anilinverbindung III vorzugsweise in einem Lösungsmittel lösen. Gegebenenfalls wird man im Anschluss an die Zugabe der Anilinverbindung III dann noch eine gewisse Zeit, z. B. 10 min. bis 10 h, insbesondere 20 min. bis 5 h, nachreagieren lassen.

Die Isolierung der Verbindung I aus der Reaktionsmischung kann in an sich bekannter Weise erfolgen. Sofern die Umsetzung in einem Lösungsmittel durchgeführt wurde, wird man in der Regel die Reaktionsmischung einengen und/oder abkühlen und/oder ein Fällungsmittel zugeben. Geeignete Fällungsmittel sind Lösungsmittel, in welchen sich die Verbindung I zumindest bei Temperaturen unterhalb 25 °C nicht oder nur in geringem Ausmaß löst. Hierzu zählen insbesondere aliphatische und cycloaliphatische Kohlenwasserstoffe wie Pentan, Hexan, Cyclohexan, Heptan, Petrolether und dergleichen. Der Fällung/Kristallisation können sich weitere Reinigungsmaßnahmen anschließen.

Das erfindungsgemäße Verfahren liefert die Verbindungen I in hoher Ausbeute von in der Regel wenigstens 80 % und häufig wenigstens 90 %, bezogen auf die Verbindung II, und guten Reinheiten bereits im Rohprodukt von in der Regel wenigstens 90 %, ohne dass aufwendige Kristallisations- oder sonstige Reinigungsmaßnahmen erforderlich sind. Selbstverständlich können weitere Reinigungsmaßnahmen, z. B. Kristallisationen in an sich bekannter Weise durchgeführt werden. Überraschenderweise liefert das erfindungsgemäße Verfahren die Verbindung I mit einem hohen E/Z-Isomerenverhältnis von E:Z > 4, was im Hinblick auf die Verwendung der Verbindungen I als Insektizid von Vorteil ist. Eine weitere Erhöhung des E/Z-Verhältnisses lässt sich in bekannter Weise durch Isomerisierung mit Iod erzielen. Die Isomerisierung des Z-Isomers von I in das E-Isomer in Gegenwart von Iod ist in der PCT/EP2004/012872 vom 12.11.2004 beschrieben, auf deren Offenbarung hiermit Bezug genommen wird.

Die im erfindungsgemäßen Verfahren eingesetzten Hydrazonverbindungen der allgemeinen Formel II können in Analogie zu den aus dem Stand der Technik bekannten Verfahren zur Herstellung von Semicarbazonen durch Umsetzung eines Phenylbenzylketons der Formel IV mit einem Hydrazid der Formel V hergestellt werden, z. B. in Analogie zu den in J. Am. Chem. Soc. 75, 1953, S. 2259-2261, J. Org. Chem. 55, 1990, S. 1070-1076 und Synthesis, 1985, S. 1048-1051 beschriebenen Methoden.

Hierzu hat es sich als vorteilhaft erwiesen, wenn man die Umsetzung des Benzylphenylketons IV mit dem Hydrazid V in Gegenwart einer Säure vornimmt. Beispiele prinzipiell geeigneter Säuren sind Schwefelsäure, organische Sulfonsäuren, insbesondere aromatische Sulfonsäuren wie p-Toluolsulfonsäure und Benzolsulfonsäure, aliphatische Sulfonsäuren wie Methansulfonsäure und Trifluormethansulfonsäure, aromatische Carbonsäuren wie Benzoesäure und substituierte Benzoesäuren wie 4-Trifluormethylbenzoesäure, weiterhin aliphatische Carbonsäuren mit vorzugsweise 1 bis 4 C-Atomen, z. B. Essigsäure und Propionsäure. Bevorzugte Säuren sind Carbonsäuren, insbesondere aliphatische Carbonsäuren mit vorzugsweise 1 bis 4 C-Atomen und speziell Essigsäure. In der Regel wird man die Säure in einer Menge von 0,01 bis 2 mol pro mol Verbindung IV und insbesondere in einer Menge von 0,05 bis 1,5 mol/mol Verbindung IV einsetzen. Im Falle der Sulfonsäuren sind katalytischen Mengen, d. h. in Mengen von weniger als 1 mol pro Mol Verbindung IV, insbesondere im Bereich von 0,01 bis 0,5 mol/mol und speziell im Bereich von 0,05 bis 0,2 mol/mol bevorzugt. Im Falle der Carbonsäuren können auch größere Mengen an Säuren eingesetzt werden, z. B. 0,1 mol bis 2 mol und speziell 0,5 bis 1,5 mol pro mol Verbindung IV.

Zur Umsetzung von Keton IV mit dem Hydrazid V wird man die Verbindungen vorzugsweise in einem Molverhältnis IV:V im Bereich von 1:2 bis 1,1:1, insbesondere von 1:1,5 bis 1:1 und besonders bevorzugt von 1:1,3 bis 1:1,05 einsetzen.

In einer bevorzugten Ausführungsform der Erfindung setzt man ein Hydrazid der Formel V ein worin R für C₁-C₄-Alkoxy und insbesondere Methoxy steht. Derartige Hydrazide V werden im Folgenden auch als Carbazat V bezeichnet.

Die Umsetzung von Keton IV mit dem Hydrazid V erfolgt vorteilhafterweise bei Temperaturen im Bereich von 10 bis 100 °C, insbesondere 20 bis 80 °C. Der Reaktionsdruck ist für den Erfolg der Umsetzung von untergeordneter Bedeutung und kann beispielsweise im Bereich von 500 mbar bis 10 bar liegen. Vorzugsweise wird die Reaktion im Bereich des Normaldrucks, d. h. im Bereich von 0,9 bis 1,2 bar durchgeführt. Die für die Umsetzung erforderliche Reaktionszeit liegt in der Regel im Bereich von 4 h bis 72 h und insbesondere im Bereich von 8 h bis 60 h.

Die Umsetzung kann grundsätzlich in Substanz durchgeführt werden. Vorzugsweise setzt man jedoch das Hydrazid der Formel V mit dem Keton IV in einem organischen Lösungsmittel um. Bevorzugte organische Lösungsmittel sind C₁-C₄-Alkanole, insbesondere Methanol und Ethanol sowie aromatische Lösungsmittel, insbesondere Alkylbenzole wie Toluol, Xylole, Ethylbenzol, Cumol (2-Propylbenzol), Cymole (Isopropyltoluole) und Mesitylen, weiterhin Chlorbenzol, 1,2-, 1,3- und 1,4-Dichlorbenzol und Mischungen dieser Lösungsmittel.

Das bei der Umsetzung des Ketons IV mit dem Hydrazid V gebildete Wasser kann in an sich bekannter Weise während der Umsetzung aus der Reaktionsmischung entfernt werden, z. B. durch Auskreisen als Azeotrop mit dem in der Reaktion eingesetzten Lösungsmittel. Das Reaktionswasser kann jedoch auch in der Reaktionsmischung verbleiben.

Zur Umsetzung können Keton IV und Hydrazid V in an sich beliebiger Weise miteinander in Kontakt gebracht werden. In der Regel wird man das Keton IV und das Hydrazid V in einem Reaktionsgefäß, gegebenenfalls zusammen mit dem gewünschten Lösungsmittel, vorlegen und dann die gewünschten Reaktionsbedingungen einstellen. Man kann jedoch auch die Hauptmenge oder Gesamtmenge an Keton IV und Hydrazid V, gegebenenfalls in einem Lösungsmittel, unter Reaktionsbedingungen in das Reaktionsgefäß eintragen oder eine der Komponenten IV oder V vorlegen und die Hauptmenge der anderen Komponente im Verlauf der Umsetzung zuführen.

Die Isolierung der Verbindung II aus der Reaktionsmischung kann in an sich bekannter Weise erfolgen. Sofern die Umsetzung in einem Lösungsmittel durchgeführt wurde, wird man in der Regel die Reaktionsmischung einengen und/oder abkühlen und/oder ein Fällungsmittel zugeben. Geeignete Fällungsmittel sind Lösungsmittel, in welchen sich die Verbindung II zumindest bei Temperaturen unterhalb 25 °C nicht oder nur in begrenztem Ausmaß löst. Hierzu zählen insbesondere aliphatische und cycloaliphatische Kohlenwasserstoffe wie Pentan, Hexan, Cyclohexan, Heptan, Petrolether, Methanol, Ethanol, Alkylbenzole und dergleichen. Der Fällung/Kristallisation können sich weitere Reinigungsmaßnahmen anschließen. Wird die Umsetzung wie bevorzugt in einem Alkohol, insbesondere in Methanol oder Ethanol oder in einem Alkylbenzol durchgeführt, ist in der Regel die Zugabe eines Fällungsmittels nicht erforderlich.

Die hier beschriebene Umsetzung des Ketons IV mit dem Hydrazid V liefert die Verbindungen II in hoher Ausbeute von in der Regel wenigstens 80 % und häufig wenigstens 95 %, bezogen auf die Verbindung II, und sehr hohen Reinheiten von häufig wenigstens 90 % und insbesondere wenigstens 95 %, ohne dass aufwendige Kristallisations- oder sonstige Reinigungsmaßnahmen erforderlich sind. Daher kann auf eine Isolierung der Verbindung II aus der Reaktionsmischung verzichtet werden.

Eine bevorzugte Ausführungsform der Erfindung betrifft daher ein Verfahren, bei der man in einem ersten Schritt das Hydrazon-Derivat der Formel II durch Umsetzung von Keton IV mit dem Hydrazid V herstellt und anschließend die Verbindung II ohne Isolierung mit der Anilinverbindung III umsetzt. Hierzu kann es von Vorteil sein, wenn man einen Teil oder die Gesamtmenge des zur Herstellung des Hydrazons II verwendeten Lösungsmittels entfernt und durch ein anderes Lösungsmittel substituiert. Insbesondere wird man jedoch die Umsetzung des Hydrazons II mit dem Anilin III in dem zur Herstellung des Hydrazons II verwendeten Lösungsmittel durchführen.

Die zur Herstellung des Hydrazons II eingesetzten Ketone IV und Verfahren zu ihrer Herstellung sind aus dem Stand der Technik, z. B. aus WO 00/18714, JP 4168826 und WO 03/091203 bekannt.

Die folgenden Beispiele dienen lediglich der Erläuterung der Erfindung und sind nicht einschränkend zu verstehen.

Die angegebenen Reinheiten und Isomerenverhältnisse wurde mittels Hochdruckflüssigchromatographie (HPLC) über die Flächenverhältnisse der jeweiligen Peaks bestimmt.

Im Zusammenhang mit den NMR-Spektren steht s für ein Singulett, d für ein Dublett und t für ein Triplett. MS steht für Massenspektrum und IR für IR-Spektrum.

### Beispiel 1: Herstellung des Hydrazons der Formel II, worin R Methoxy bedeutet, R¹ für 3-CF₃ und R² für 4-CN steht

### Variante A:

18,8 g (0,21 mol) Methylcarbazat (HN-NH-C(O)-OCH₃) und 57,8 g (0,20 mol) 3-Trifluormethylphenyl-4-cyanobenzylketon (Verbindung IV mit R¹ = 3-CF₃ und R² = 4-CN) wurden in 700 ml Methanol bei 20 °C gelöst. Anschließend gab man 2 ml konzentrierte Schwefelsäure zu, rührte 2 Tage bei 20 °C und isolierte den ausgefallenen Feststoff. Dieser wurde mit 100 ml Methanol gewaschen und im Trockenschrank bei 50 °C / 10 mbar getrocknet. Man erhielt auf diese Weise 62,6 g (entsprechend einer Ausbeute von 86,7 % der Theorie) des Hydrazons II {R¹ = 3-CF₃, R² = 4-CN} mit einer Reinheit (HPLC) von 99,6 %.
Schmelzpunkt: 171 °C
MS (EI): m/e = 361 (M⁺-Ion)
IR: 2245 cm⁻¹ (CN); 1704 cm⁻¹ (C=O)
- ¹H-NMR (DMSO): δ/ppm =: 3,8 (s, 3H); 4,5 (s, 2H); 7,4 (d, 2H); 7,64 (t, 1H); 7,7 (d, 1H), 7,8 (d, 2H); 8,0 (d, 1 H); 8,15 (s, 1H); 10,95 (s, 1 H)
- ¹³C-NMR (DMSO): δ/ppm =: 31,77 (t); 52,19 (q); 109,30 (s); 118,64 (s); 122,51 (d); 124,01 (s; Kopplungskonst. C/F: 272,3 Hz); 125,30 (d); 129,16 (d, 2C); 129,24 (s); 129,61 (d); 130.25 (d); 132,51 (d, 2C); 138,07 (s); 142,11 (s); 146,45 (s); 154,58 (s)

### Variante B:

7,3 g (0,025 mol) 3-Trifluormethylphenyl-4-cyanobenzylketon und 2,4 g (0,025 mol) Methylcarbazat (97%ig) wurden in Gegenwart von 2,0 g Essigsäure in 50 g Xylol 24 h bei 50°C umgesetzt. Nach Abkühlen auf 20°C wurde der ausgefallene Feststoff abgetrennt mit 10 g Xylol gewaschen und im Trockenschrank bei 50 °C / 10 mbar getrocknet. Man erhielt auf diese Weise 8,0 g (entsprechend einer Ausbeute von 88,1 % der Theorie) des Hydrazons II {R¹ = 3-CF₃, R² = 4-CN} mit einer Reinheit (HPLC) von 99,9 %.

### Beispiel 2: Herstellung der Verbindung I, worin R¹ für 3-CF₃ und R² für 4-CN stehen und R³ 4-OCF₃ bedeutet

### Variante A:

In einem Reaktionsgefäß mit Destillationskolonne wurden 7,2 g (0,02 mol) des Hydrazons aus Beispiel 1 und 3,9 g (0,022 mol) 4-Trifluormethoxyanilin in 100 g Xylol zusammengegeben und die Mischung wurde zum Rückfluss erhitzt. Innerhalb 7 h wurden mit einem hohen Rücklaufverhältnis 80 g einer Mischung aus Methanol und Xylol abdestilliert. Man kühlte die Reaktionsmischung langsam auf 60 °C ab und gab bei dieser Temperatur 5 g Cyclohexan zu. Anschließend kühlte man die Mischung weiter auf 10 °C ab. Der ausgefallene Feststoff wurde abgetrennt, mit 10 g Cyclohexan gewaschen und im Trockenschrank bei 80 °C / 10 mbar getrocknet. Man erhielt auf diese Weise 9,4 g der Titelverbindung (entsprechend einer Ausbeute von 92,2 % der Theorie) in Form eines Isomerengemischs mit einer Reinheit von 98,1 % (81,2 % E-Isomer und 16,9 % Z-Isomer).

### Variante B:

In einem Reaktionsgefäß mit Destillationskolonne wurden 21,6 g (0,06 mol) des Hydrazons aus Beispiel 1 und 11,7g (0,066 mol) 4-Trifluormethoxyanilin in 300 g Xylol zusammengegeben und die Mischung wurde zum Rückfluss erhitzt. Innerhalb 7 h wurden mit einem hohen Rücklaufverhältnis 12 g einer Mischung aus Methanol und Xylol abdestilliert. Zur Kristallisation des Produkts wurden weitere 234 g Xylol abdestilliert. Man kühlte langsam auf 60 °C ab, gab bei dieser Temperatur 75 g Cyclohexan zu und kühlte anschließend weiter auf 10 °C ab. Der ausgefallene Feststoff wurde abgetrennt, mit 30 g Cyclohexan gewaschen und im Trockenschrank bei 100 °C / 10 mbar getrocknet. Man erhielt auf diese Weise 28,0 g der Titelverbindung (entsprechend einer Ausbeute von 84,6 % der Theorie) in Form eines Isomerengemischs mit einer Reinheit von 91,5 % (77,4 % E-Isomer und 14,1 % Z-Isomer).

## Patentansprüche

1. Verfahren zur Herstellung von Phenylsemicarbazon-Verbindungen der Formel I 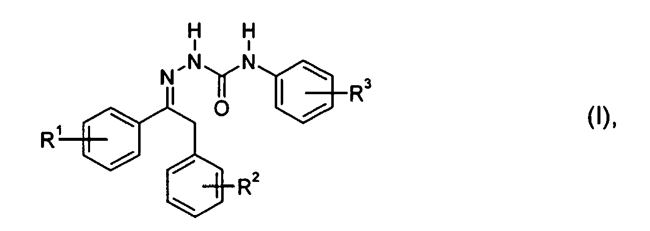 worin R¹ und R² unabhängig voneinander für Wasserstoff, Halogen, CN, C₁-C₄-Alkyl, C₁-C₄-Alkoxy, C₁-C₄-Haloalkyl oder C₁-C₄-Haloalkoxy stehen und R³ für C₁-C₄-Alkoxy, C₁-C₄-Haloalkyl oder C₁-C₄-Haloalkoxy steht,
umfassend die Umsetzung einer Hydrazonverbindung der allgemeinen Formel II, worin R für C₁-C₄-Alkoxy, Amino, C₁-C₄-Alkylamino oder Di-(C₁-C₄-alkyl)amino steht und R¹, R² die zuvor genannten Bedeutungen haben, 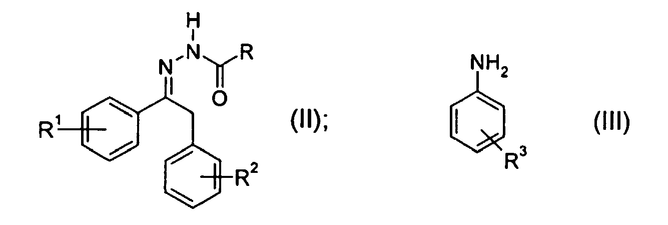 mit einer Anilinverbindung der allgemeinen Formel III, worin R³ die zuvor genannte Bedeutung hat.

2. Verfahren nach Anspruch 1, wobei R für C₁-C₄-Alkoxy steht.

3. Verfahren nach Anspruch 2, wobei R für Methoxy steht.

4. Verfahren nach Anspruch 1, wobei R für Amino, Methylamino, Ethylamino oder Dimethylamino steht.

5. Verfahren nach einem der vorhergehenden Ansprüche, wobei die Anilinverbindung III und Hydrazonverbindung der allgemeinen Formel II in einem Molverhältnis II:III im 1:1,5 bis 1,5:1 eingesetzt wird.

6. Verfahren nach einem der vorhergehenden Ansprüche, wobei man die Hydrazonverbindung der Formel II durch Umsetzung eines Benzylphenylketons der Formel IV 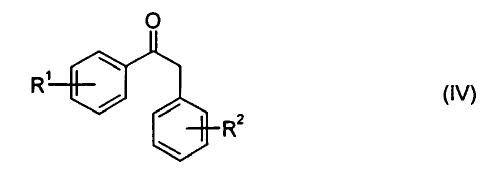 worin R¹, R² die zuvor genannten Bedeutungen aufweisen, mit einem Hydrazid der Formel V 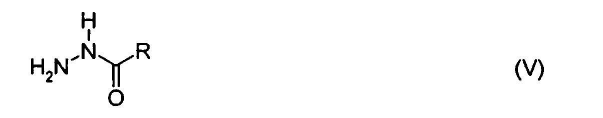 worin R die zuvor genannte Bedeutung hat, bereitstellt.

7. Verfahren nach Anspruch 6, wobei R in Formel V für C₁-C₄-Alkoxy steht.

8. Verfahren nach Anspruch 7, wobei man die Umsetzung des Benzylphenylketons IV mit dem Hydrazid V in Gegenwart einer Säure vornimmt.

9. Verfahren nach einem der Ansprüche 6 bis 8, wobei man die Hydrazonverbindung der allgemeinen Formel II ohne Isolierung mit der Anilinverbindung III umsetzt.

10. Verfahren nach einem der vorhergehenden Ansprüche, wobei R¹ in den Formeln I, II und IV meta-Trifluormethyl bedeutet, R² in den Formeln I, II und IV für para-CN steht und R³ in den Formeln I und III für para-Trifluormethoxy steht.

11. Hydrazonverbindungen der allgemeinen Formel II 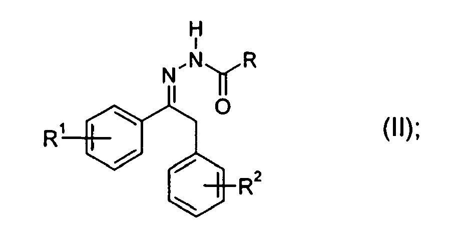 worin R¹ für Wasserstoff, Halogen, CN, C₁-C₄-Alkyl C₁-C₄-Alkoxy, C₁-C₄-Haloalkyl oder C₁-C₄-Haloalkoxy steht, R² für CN steht und R für C₁-C₄-Alkoxy, Amino oder C₁-C₄-Alkylamino oder Di-(C₁-C₄-alkyl)amino steht.

12. Hydrazonverbindungen der allgemeinen Formel II nach Anspruch 11, wobei R für C₁-C₄-Alkoxy steht.

13. Hydrazonverbindungen der allgemeinen Formel II nach Anspruch 12, wobei R für Methoxy steht.

14. Hydrazonverbindungen der allgemeinen Formel II nach einem der Ansprüche 11 bis 13, wobei R¹ meta-Trifluormethyl bedeutet und R² für para-CN steht.

## Claims

1. A process for preparing phenylsemicarbazone compounds of the formula I 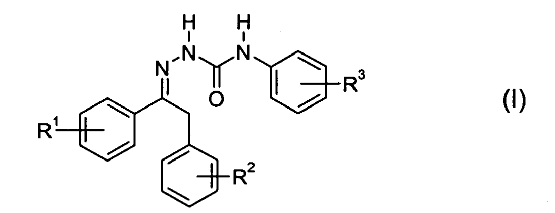 where R¹ and R² are each independently hydrogen, halogen, CN, C₁-C₄-alkyl, C₁-C₄-alkoxy, C₁-C₄-haloalkyl or C₁-C₄-haloalkoxy, and R³ is C₁-C₄-alkoxy, C₁-C₄-haloalkyl or C₁-C₄-haloalkoxy,
comprising the reaction of a hydrazone compound of the general formula II where R is C₁-C₄-alkoxy, amino, C₁-C₄-alkylamino or di(C₁-C₄-alkyl)amino, and R¹, R² are each as defined above 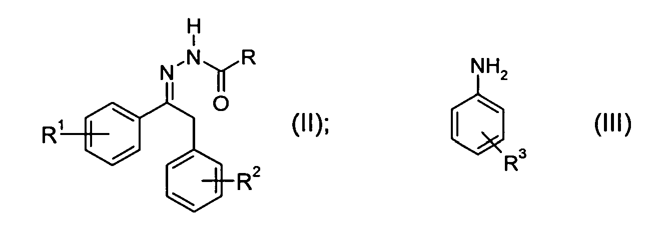 with an aniline compound of the general formula III where R³ is as defined above.

2. The process according to claim 1, wherein R is C₁-C₄-alkoxy.

3. The process according to claim 2, wherein R is methoxy.

4. The process according to claim 1, wherein R is amino, methylamino, ethylamino or dimethylamino.

5. The process according to any of the preceding claims, wherein the aniline compound III and hydrazone compound of the general formula II are used in a molar II:III ratio of from 1:1.5 to 1.5:1.

6. The process according to any of the preceding claims, wherein the hydrazone compound of the formula II is provided by reacting a benzyl phenyl ketone of the formula IV 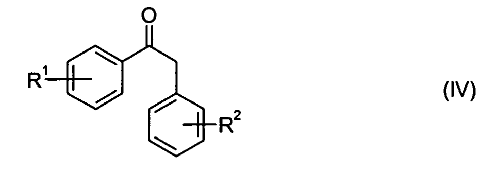 where R¹, R² are each as defined above, with a hydrazide of the formula V 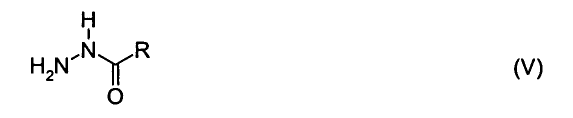 where R is as defined above.

7. The process according to claim 6, wherein R in formula V is C₁-C₄-alkoxy.

8. The process according to claim 7, wherein the reaction of the benzyl phenyl ketone IV with the hydrazide V is undertaken in the presence of an acid.

9. The process according to any of claims 6 to 8, wherein the hydrazone compound of the general formula II is reacted with the aniline compound III without isolation.

10. The process according to any of the preceding claims, wherein R¹ in the formulae I, II and IV is meta-trifluoromethyl, R² in the formulae I, II and IV is para-CN and R³ in the formulae I and III is para-trifluoromethoxy.

11. A hydrazone compound of the general formula II 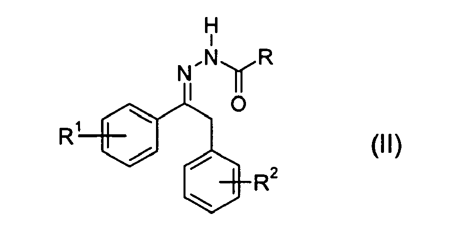 where R¹ is hydrogen, halogen, CN, C₁-C₄-alkyl, C₁-C₄-alkoxy, C₁-C₄-haloalkyl or C₁-C₄-haloalkoxy, R² is CN and R is C₁-C₄-alkoxy, amino or C₁-C₄-alkylamino or di(C₁-C₄-alkyl)amino.

12. The hydrazone compound of the general formula II according to claim 11, wherein R is C₁-C₄-alkoxy.

13. The hydrazone compound of the general formula II according to claim 12, wherein R is methoxy.

14. The hydrazone compound of the general formula II according to any of claims 11 to 13, wherein R¹ is meta-trifluoromethyl and R² is para-CN.

## Revendications

1. Procédé pour la préparation de composés de type phénylsemicarbazone de formule I, 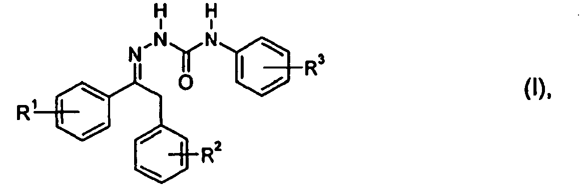 dans laquelle R¹ et R² représentent, indépendamment l'un de l'autre, hydrogène, halogène, CN, alkyle en C₁-C₄, alcoxy en C₁-C₄, halogénoalkyle en C₁-C₄ ou halogénoalcoxy en C₁-C₄ et R³ représente alcoxy en C₁-C₄, halogénoalkyle en C₁-C₄ ou halogénoalcoxy en C₁-C₄, comprenant la transformation d'un composé de type hydrazone de formule générale II, où R représente alcoxy en C₁-C₄, amino, (alkyle en C₁-C₄) amino ou di-(alkyle en C₁-C₄) amino et R¹, R² ont les significations susmentionnées, 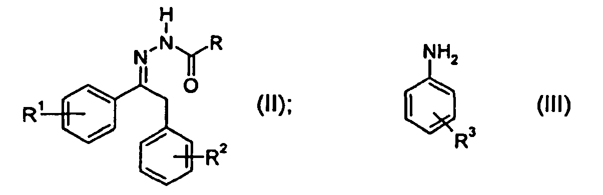 avec un composé de type aniline de formule générale III, dans laquelle R³ a la signification susmentionnée.

2. Procédé selon la revendication 1, où R représente alcoxy en C₁-C₄.

3. Procédé selon la revendication 2, où R représente méthoxy.

4. Procédé selon la revendication 1, où R représente amino, méthylamino, éthylamino ou diméthylamino.

5. Procédé selon l'une quelconque des revendications précédentes, où le composé de type aniline III et le composé de type hydrazone de formule générale II sont utilisés dans un rapport molaire II:III de 1:1,5 à 1,5:1.

6. Procédé selon l'une quelconque des revendications précédentes, dans lequel on prépare le composé de type hydrazone de formule II par transformation d'une benzylphénylcétone de formule IV 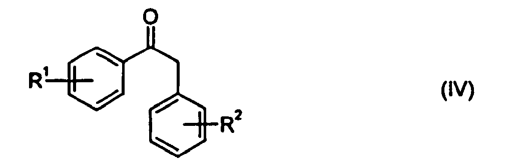 dans laquelle R¹, R² présentent les significations susmentionnées avec un hydrazide de formule V 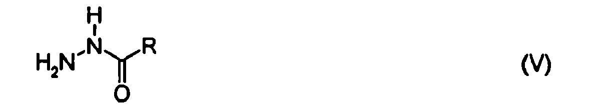 dans laquelle R a la signification susmentionnée.

7. Procédé selon la revendication 6, où R dans la formule V représente alcoxy en C₁-C₄.

8. Procédé selon la revendication 7, dans lequel on réalise la transformation de la benzylphénylcétone IV avec l'hydrazide V en présence d'un acide.

9. Procédé selon l'une quelconque des revendications 6 à 8, où on transforme le composé hydrazone de formule générale II sans isolement avec le composé de type aniline III.

10. Procédé selon l'une quelconque des revendications précédentes, dans lequel R¹ dans les formules I, II et IV signifie méta-trifluorométhyle, R² dans les formules I, II et IV représente para-CN et R³ dans les formules I et III représente para-trifluorométhoxy.

11. Composés de type hydrazone de formule générale II 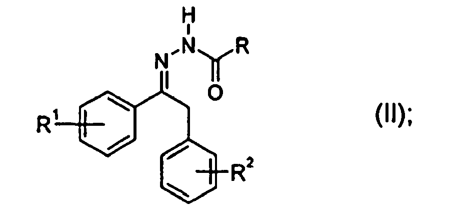 dans laquelle R¹ représente hydrogène, halogène, CN, alkyle en C₁-C₄, alcoxy en C₁-C₄, halogénoalkyle en C₁-C₄ ou halogénoalcoxy en C₁-C₄, R² représente CN et R représente alcoxy en C₁-C₄, amino ou (alkyle en C₁-C₄) amino ou di- (alkyle en C₁-C₄) amino.

12. Composés de type hydrazone de formule générale II selon la revendication 11, où R représente alcoxy en C₁-C₄.

13. Composés de type hydrazone de formule générale II selon la revendication 12, où R représente méthoxy.

14. Composés de type hydrazone de formule générale II selon l'une quelconque des revendications 11 à 13, où R¹ signifie méta-trifluorométhyle et R² représente para-CN.
